# EUROPEAN PATENT APPLICATION

(11) **EP 4 219 444 A1**
(43) Date of publication of application: **02.08.2023**
(21) Application number: 21872272.6
(22) Date of filing: 14.09.2021
(51) Int. Cl.: C07C 209/14, C07C 217/08, C07B 61/00

(54) **METHOD FOR PRODUCING TERTIARY AMINE COMPOSITION**

(30) Priority: 25.09.2020 JP 2020161042
(71) Applicant: Kao Corporation, Chuo-ku Tokyo 103-8210 (JP)
(72) Inventor: HIRAYAMA, Arata, Wakayama-shi, Wakayama 640-8580 (JP); KOBAYASHI, Takeshi, Wakayama-shi, Wakayama 640-8580 (JP); TATENO, Gosuke, Wakayama-shi, Wakayama 640-8580 (JP); MYRDEK, Thomas, 46446 Emmerich am Rhein (DE)
(74) Representative: Hoffmann Eitle
(86) International application number: PCT/JP2021/033817
(87) International publication number: WO 2022/065142

(57) **Abstract**

The present invention provides a method for producing a tertiary amine composition, said method comprising the steps (1) to (3) described below. Step (1): an amination reaction step wherein dimethyl amine and an aliphatic aldehyde or an aliphatic alcohol having from 8 to 36 carbon atoms are reacted with each other in the presence of a metal catalyst Step (2): a distillation step wherein a reaction liquid obtained through the step (1) is separated into a concentrate and an evaporation product that contains a low-boiling-point compound by means of distillation Step (3): a centrifugal separation step wherein the concentrate obtained through the step (2) is separated into a sediment and a liquid layer that contains a tertiary amine composition by means of a centrifugal force

## Description

### Field of the Invention

The present invention relates to a method for producing a tertiary amine composition.

### Background of the Invention

Tertiary amines are an important intermediate material in domestic and industrial application fields. Quaternary ammonium salts obtained by conversion of the tertiary amines to quaternary ammoniums are used in various applications such as antirust agents, bactericides, fabric softeners, shampoos, rinses, antistatic agents, detergents, dispersants, and textile auxiliaries.

Various methods for producing a tertiary amine are known. Typical examples of the methods include a technique in which an alcohol is subjected to an amination with a secondary amine as raw materials in the presence of a metal catalyst to produce a dimethylalkylamine as a main product (JPS61-15865A (PTL 1)).

It is known that by a method for producing a tertiary amine described in PTL 1, a dimethylalkylamine is obtained as a main product and a methyldialkylamine is further obtained as a product.

The methods for producing a tertiary amine have often been investigated to obtain the tertiary amine at a high yield. Recently, improvement in hue of the obtained tertiary amine resulting in an increase in product value is required.

For example, JP2011-256245A (PTL 2) discloses a method for purifying transesterified oil and fat including adding a calcium salt and/or a magnesium salt, and water to transesterified oil and fat obtained by transesterification using a sodium compound as a catalyst to deposit a water-insoluble calcium soap and/or magnesium soap, removing water to further deposit a sodium salt, and removing the calcium soap and/or the magnesium soap, and the sodium salt by filtration and/or centrifugation.

In PTL 2, the calcium soap, the magnesium soap, and the sodium salt are removed by filtration and/or centrifugation to achieve a high yield of transesterified oil and fat and favorable color tone.

### Summary of the Invention

The present invention relates to a method for producing a tertiary amine composition including the following steps (1) to (3).
Step (1): an amination step of reacting an aliphatic alcohol or an aliphatic aldehyde having 8 or more and 36 or less carbon atoms with dimethylamine in the presence of a metal catalyst;
Step (2): a distillation step of separating a reaction liquid obtained through the step (1) by distillation into a distilled product containing a low-boiling-point compound and a concentrate; and
Step (3): a centrifugation step of separating the concentrate obtained through the step (2) by a centrifugal force into a precipitate and a liquid phase containing the tertiary amine composition.

### Brief Description of the Drawing

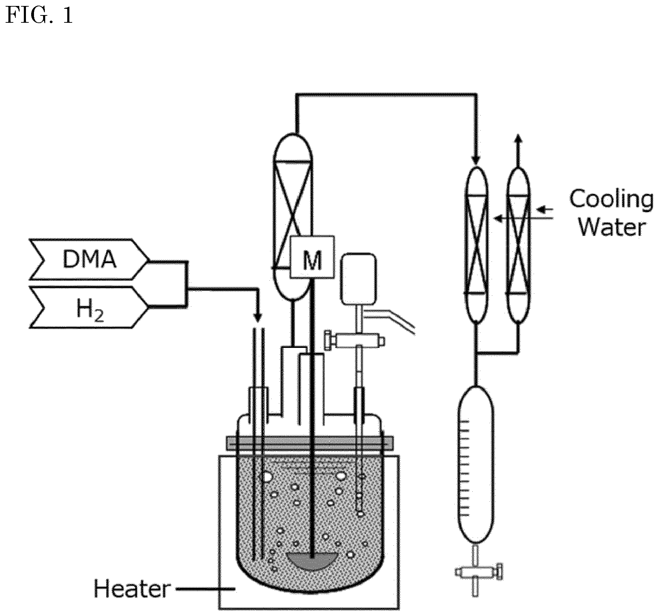

FIG. 1 is a schematic view of a stirring vessel-type reactor used in Examples.

### Detailed Description of the Invention

Although an invention in PTL 2 is made to achieve a favorable color tone, the invention is only a technique for achieving the favorable color tone by the method for purifying transesterified oil and fat and is not a technique that can be adopted for the method for producing a tertiary amine composition. For this reason, the invention may be improved.

In contrast, PTL 1 discloses the method for producing a tertiary amine using a metal catalyst. A product obtained by the method for producing a tertiary amine in PTL 1 contains a colored substance due to elution of the metal catalyst and the like, and the hue of the product may be deteriorated. Therefore, for the method for producing a tertiary amine, a technique for improving the hue to increase a product value is required.

Accordingly, the present invention relates to a method for producing a tertiary amine composition having a favorable hue (in particular, a favorable color tone) by suitably removing a low-boiling-point compound and a colored substance.

The present inventors have extensively investigated a production method for obtaining a tertiary amine composition having a favorable hue, and found that a method for producing a tertiary amine composition having a favorable hue (in particular, a favorable color tone) by suitably removing a low-boiling-point compound and a colored substance can be provided.

Specifically, the present invention relates to a method for producing a tertiary amine composition including the following steps (1) to (3).

Step (1): an amination step of reacting an aliphatic alcohol or an aliphatic aldehyde having 8 or more and 36 or less carbon atoms with dimethylamine in the presence of a metal catalyst;

Step (2): a distillation step of separating a reaction liquid obtained through the step (1) by distillation into a distilled product containing a low-boiling-point compound and a concentrate; and

Step (3): a centrifugation step of separating the concentrate obtained through the step (2) by a centrifugal force into a precipitate and a liquid phase containing the tertiary amine composition.

The present invention can provide the method for producing a tertiary amine composition having a favorable hue (in particular, a favorable color tone) by suitably removing the low-boiling-point compound and a colored substance.

The method for producing a tertiary amine composition according to the present invention includes the following steps (1) to (3).
Step (1): an amination step of reacting an aliphatic alcohol or an aliphatic aldehyde having 8 or more and 36 or less carbon atoms with dimethylamine in the presence of a metal catalyst;
Step (2): a distillation step of separating a reaction liquid obtained through the step (1) by distillation into a distilled product containing a low-boiling-point compound and a concentrate; and
Step (3): a centrifugation step of separating the concentrate obtained through the step (2) by a centrifugal force into a precipitate and a liquid phase containing the tertiary amine composition.

Thus, the present invention can provide the method for producing a tertiary amine composition having a favorable hue (in particular, a favorable color tone) by suitably removing the low-boiling-point compound and a colored substance.

As the "colored substance" used herein, a colored substance derived from elution of the metal catalyst used in the amination, a substance derived from an eluted metal catalyst component that is colored by heat histories in the amination step and the distillation step, a substance derived from the raw materials and the product that are colored by heat histories in the amination step and the distillation step, and the like are considered.

As a general procedure for removing the colored substance, addition of a colored substance adsorbent such as activated carbon, activated clay, and diatomaceous earth, and the like are considered.

On the other hand, it is considered that in a procedure of the present invention, the eluted metal catalyst is reduced to deposit metal granules that are removable in the centrifugation step. The substance derived from the colored raw materials and product is adsorbed to the metal granules. Thus, it is considered that the colored substance is effectively removed in the centrifugation step without adding the colored substance adsorbent and the hue can be improved. Further, it is considered that the concentration of the colored substance in the concentrate can be increased by removing the low-boiling-point compound in the distillation step and the adsorption can be efficiently achieved.

### [Step (1): Amination Step]

In the amination step of the present invention, an aliphatic alcohol or an aliphatic aldehyde having 8 or more and 36 or less carbon atoms is reacted with dimethylamine in the presence of a metal catalyst.

In the amination step of the present invention, it is preferable that the reaction be performed in the presence of hydrogen from the viewpoint of a hue and reactivity.

From the viewpoint of hue and removing the metal catalyst, it is preferable that the method include a filtration step after the amination step and before the step (2): distillation step described below.

When in the amination step of the present invention, an alcohol is used as a starting material, an aliphatic alcohol having 8 or more and 36 or less carbon atoms (raw alcohol) is first dehydrogenated in the presence of the metal catalyst to produce an aldehyde, and this aldehyde comes into contact with dimethylamine (raw amine) in a reaction liquid to produce an enamine a. Subsequently, the enamine a produced in the reaction liquid is hydrogenated to obtain a "dimethylalkylamine (RN(CH₃)₂)". Monomethylamine produced from dimethylamine (raw amine) by disproportionation comes into contact with the aldehyde in the reaction liquid to produce an enamine b. The enamine b produced in the reaction liquid is then hydrogenated to produce a "methylalkylamine (RNH(CH₃))", and the "methylalkylamine (RNH(CH₃))" comes into contact with the aldehyde in the reaction liquid to produce an enamine c. Next, the enamine c produced in the reaction liquid is hydrogenated to obtain a "methyldialkylamine (R₂N(CH₃))".

Meanwhile, an aldehyde itself may also be used as the starting material of the amination without using an alcohol.

When as the alcohol or the aldehyde that is the starting material, a mixture of alcohols having different hydrocarbon groups or a mixture of aldehydes having different hydrocarbon groups is used, two hydrocarbon groups (R's) of the methyldialkylamine (R₂N(CH₃))" may be different from each other.

Herein, R is a hydrocarbon group derived from the aliphatic alcohol or the aliphatic aldehyde, and the number of carbon atoms in the hydrocarbon group is 8 or more and 36 or less.

A reaction method used in the amination is not particularly limited, and may be a suspension bed batch method, a fixed bed flowing method, or a fixed bed batch method. The reaction method may be appropriately selected according to a catalytic activity, a reaction scale, and the like. In particular, a suspension bed method is preferred from the viewpoint of hue improvement effect of the present invention.

A reactor used in the amination is not particularly limited, and for example, a stirring vessel-type reactor shown in FIG. 1 can be used. When the stirring vessel-type reactor shown in FIG. 1 is used, the raw alcohol or the raw aldehyde is first placed into a reaction vessel, the metal catalyst is added, and the inside in the reaction system is replaced by inert gas while the reaction liquid in the reaction vessel is stirred.

After the inside in the system is replaced by inert gas, the supply of hydrogen gas to the reaction vessel is started, and the reaction liquid in the reaction vessel is stirred, heated until the temperature of the reaction liquid is a predetermined temperature, and held at the predetermined temperature for a predetermined time. The supply of the raw amine to the reaction vessel is started, and the temperature of the reaction liquid is gradually increased and maintained at a predetermined temperature. Thus, the amination can be performed.

The amination is performed until the amount of an unreacted raw alcohol or an unreacted raw aldehyde in the reaction liquid is equal to or smaller than a predetermined amount. The metal catalyst contained in the reaction liquid can be removed by filtration.

The raw alcohol, the raw aldehyde, the raw amine, and the metal catalyst used in the amination are as follows.

### <Raw Alcohol and Raw Aldehyde>

The raw alcohol or the raw aldehyde used in the amination step is an aliphatic alcohol or an aliphatic aldehyde having 8 or more and 36 or less carbon atoms. In particular, the aliphatic alcohol is preferred from the viewpoint of availability.

The hydrocarbon group of the aliphatic alcohol or the aliphatic aldehyde may be a linear, branched, or cyclic hydrocarbon group, or a saturated or unsaturated hydrocarbon group.

The number of carbon atoms in the aliphatic alcohol or the aliphatic aldehyde is preferably 8 or more, more preferably 10 or more, and further preferably 12 or more, and preferably 30 or less, more preferably 26 or less, and further preferably 22 or less from the viewpoint of reactivity.

### <Raw Amine>

The raw amine used in the amination is dimethylamine.

The raw amine may be continuously supplied or intermittently supplied to the reaction vessel. It is preferable that the supply of the raw amine be appropriately controlled according to the progress of the amination.

When the raw amine is continuously supplied to the reaction vessel, the amount of the raw amine supplied is preferably 5 L/hr or more, more preferably 7 L/hr or more, and further preferably 9 L/hr or more, and preferably 100 L/hr or less, more preferably 95 L/hr or less, and further preferably 90 L/hr or less, relative to 1 kg of the raw alcohol or the raw aldehyde.

Meanwhile, the raw amine may be gaseous.

### <Metal Catalyst>

The metal catalyst used in the amination is not particularly limited, and for example, a transition metal catalyst such as a copper-based catalyst or a nickel-based catalyst, or a noble metal catalyst such as a ruthenium-based catalyst can be used.

Examples of the copper-based catalyst include catalysts described in JP H2-233A (catalysts including Cu, one or more transition metals selected from Cr, Mu, Fe, Ni, Co, and Zn, a platinum group element, and an alkali metal or alkali earth metal such as Li and Mg), catalysts described in JPH2-234A (catalysts including Cu, a fourth period transition metal element such as Ni and Co, a platinum group element, and a fourth component such as Al), and catalysts described in JP2001-151733A (catalysts including Cu, a fourth period transition metal, and a platinum group element).

Examples of the nickel-based catalyst include catalysts described in JPS50-30804A (catalysts including Ni, Cu, and Cr), catalysts described in JPH7-69999A (catalysts including Ni), catalysts described in JP2005-527516A (catalysts including Ni, Cu, Co and ZrO₂), and catalysts described in JP2007-176889A (catalysts including Ni, Cu, and an alkali metal).

Examples of the ruthenium-based catalyst include catalysts described in JPH8-243392A (catalysts including Ru and a porous oxide), catalysts described in EP729785A1 (catalysts including Ru and a noble metal), catalysts described in JP2008-150312A (catalysts including Ru, and a ZrO₂ composite oxide and/or ZrO₂ surface-treated with a metal), catalysts described in JP2007-176891A (catalysts including Ru and a porous oxide), catalysts described in JP2007-176892A (catalysts including Ru, one or more metal components selected from Ni and Co, and one or more metal components selected from the group La, Y, Mg, and Ba), and catalysts described in US4912260A (catalysts including Ru, Ni, and one or more metal components selected from Pd, Re, and Ir).

From the viewpoint of reactivity, the metal catalyst is preferably a catalyst containing at least one metal element selected from the group consisting of copper, nickel, cobalt, iron, ruthenium, platinum, rhodium, palladium, molybdenum, tungsten, and rhenium as a main active component.

When the metal catalyst contains copper as a main active component and another main active component, it is preferable that the metal catalyst contain at least one metal element selected from the group consisting of (i) a fourth period transition metal; (ii) platinum and a fifth period transition metal; and (iii) an alkali metal and an alkali earth metal as the other main active component and satisfy any one of the following conditions (a) to (c). Meanwhile, ratios in the conditions (a) to (c) represent a molar ratio between metals.
(i) The fourth period transition metal: at least one metal element selected from nickel, cobalt, iron, chromium, and zinc;
(ii) Platinum and the fifth period transition metal: at least one metal element selected from platinum, palladium, ruthenium, and rhodium;
(iii) An alkali metal and an alkali earth metal: at least one metal element selected from lithium, sodium, potassium, rubidium, cesium, magnesium, calcium, strontium, and barium.

- Condition (a): the ratio copper/fourth period transition metal is 1/9 to 99/1, and preferably 50/50 to 99/1;
- Condition (b): the ratio platinum and fifth period transition metal/(copper + fourth period transition metal) is 0 to 0.1, and preferably 0 to 0.05; and
- Condition (c): the ratio fourth period transition metal/(alkali metal + alkali earth metal) is 1/0 to 1/2, and preferably 1/0 to 1/1.

Among the metal catalysts, catalysts including copper and the fourth period transition metal (in particular, nickel), catalysts including copper, and platinum or the fifth period transition metal (in particular, ruthenium), and catalysts including copper, the fourth period transition metal (in particular, nickel), and the fifth period transition metal (in particular, ruthenium) are preferred. In particular, catalysts including copper and the fourth period transition metal (in particular, nickel) are more preferred.

The metal catalyst may be used in a form in which the main active components are supported on a porous carrier such as a metal oxide and a composite oxide. The shape of the catalyst is not particularly limited, and may be a powder shape, a spherical shape, a cylindrical shape (pellet-like shape), or a film shape.

The metal catalysts may be used alone or in combination of any two or more thereof.

The metal catalyst may be a reduced metal catalyst or a non-reduced metal catalyst. From the viewpoints of reactivity, the metal catalyst is preferably a reduced metal catalyst. The reduced metal catalyst may be prepared by reducing the metal catalyst in a reducing atmosphere such as a hydrogen gas. Therefore, the reduced metal catalyst may also be prepared, for example, by a step in which the metal catalyst in an unreduced state is placed with the raw alcohol into the reaction vessel and the reaction liquid is heated to the reaction temperature while a hydrogen gas is introduced into the reaction vessel.

It is preferable that the amount of the metal catalyst used be appropriately adjusted according to the reaction method. From the viewpoint of a hue and reactivity, the amount of the metal catalyst used is preferably 0.05 parts by mass or more, and more preferably 0.1 parts by mass or more, and preferably 10 parts by mass or less, and more preferably 1 part by mass or less, relative to the whole amount of the raw alcohol or the raw aldehyde.

The reaction method is not particularly limited. Examples of the reaction method include a suspension bed method, a fixed-bed batch method, a fixed-bed continuous method, and a combination thereof. In particular, a suspension bed method is preferred from the viewpoint of hue improvement effect of the present invention.

### <Reaction Condition of Amination>

In the amination step, the reaction temperature before the supply of the raw amine (dimethylamine) after the supply of the hydrogen gas to the reaction vessel (reaction liquid) is appropriately determined in terms of the boiling point of the raw alcohol. From the viewpoint of a hue and reactivity, the reaction temperature is preferably 150°C or higher, more preferably 160°C or higher, and further preferably 170°C or higher, and preferably 220°C or lower, more preferably 210°C or lower, and further preferably 200°C or lower.

The reaction temperature after the supply of the raw amine to the reaction vessel (reaction liquid) is appropriately determined in terms of the boiling point of the raw alcohol. From the viewpoint of a hue and reactivity, the reaction temperature is preferably 160°C or higher, more preferably 170°C or higher, and further preferably 180°C or higher, and preferably 300°C or lower, more preferably 250°C or lower, and further preferably 230°C or lower.

In the amination step, a portion of the reaction liquid in the reaction vessel is appropriately sampled with time, and the introduction of the raw amine is terminated at the time point when the amount of the unreacted raw alcohol or the unreacted raw aldehyde in the reaction liquid is 1.5% by mass or less. The amount at the time point is a reaction end point.

The unreacted raw alcohol or the unreacted raw aldehyde can be quantified by a gas chromatography through a method described in Examples.

The amount of the hydrogen gas supplied is preferably 15 L/hr or more, and more preferably 20 L/hr or more, and preferably 45 L/hr or less, more preferably 40 L/hr or less, and further preferably 35 L/hr or less, relative to 1 kg of the raw alcohol or the raw aldehyde.

### [Step (2): Distillation Step]

In the distillation step of the present invention, the reaction liquid obtained through the amination step is separated by distillation into a distilled product containing a low-boiling-point compound and a concentrate.

The "low-boiling-point compound" used herein is appropriately determined in terms of the boiling point of the raw alcohol. For example, when the number of carbon atoms is 12, the low-boiling-point compound refers to a compound having a boiling point of 280°C or lower.

In the distillation step of the present invention, the reaction liquid obtained through the amination step is placed into a container such as a flask, and distillation and separation operations that separate the reaction liquid into a distilled product containing a low-boiling-point compound and a concentrate are performed using a distiller under a predetermined distillation condition.

### <Distillation Condition>

From the viewpoint of a hue and distillation efficiency, the distillation temperature is preferably 70°C or higher, more preferably 90°C or higher, and further preferably 110°C or higher, and preferably 300°C or lower, more preferably 250°C or lower, and further preferably 200°C or lower.

The degree of vacuum is appropriately adjusted according to the distillation temperature and is preferably 0.01 kPa or more, more preferably 0.05 kPa or more, and further preferably 0.1 kPa or more, and preferably 5 kPa or less, more preferably 3 kPa or less, and further preferably 1 kPa or less.

The concentrate obtained through the distillation step contains a dialkylmethylamine represented by the following formula (I):

Formula (I): R₂N(CH₃)

wherein R is a hydrocarbon group derived from the aliphatic alcohol or the aliphatic aldehyde and the number of carbon atoms in the hydrocarbon group is 8 or more and 36 or less, in an amount of preferably 50% by mass or more, more preferably 70% by mass or more, and further preferably 75% by mass or more.

A portion of the concentrate collected through the distillation step is sampled, and the concentrate obtained through the distillation step can be quantified and quantitatively determined by a gas chromatography through a method described in Examples.

The concentrate obtained by distillation and separation through the distillation step contains an ester compound in an amount of preferably 1% by mass or more, more preferably 3% by mass or more, and further preferably 5% by mass or more, and preferably 20% by mass or less, more preferably 18% by mass or less, and further preferably 15% by mass or less.

The ester compound used herein refers to an ester compound produced from a fatty acid that is a saturated or unsaturated aliphatic carboxylic acid having 8 or more and 36 or less carbon atoms and an alcohol having 8 or more and 36 or less carbon atoms, and in particular, refers to a fatty-acid monoester compound having 8 or more and 36 or less carbon atoms that is produced from a monovalent fatty acid that is a saturated or unsaturated aliphatic carboxylic acid and a monohydric alcohol.

In contrast, the distilled product obtained by distillation and separation through the distillation step contains as the low-boiling-point compound an alkyldimethylamine represented by the following formula (II):

Formula (II): RN(CH₃)₂

wherein R is a hydrocarbon group derived from the aliphatic alcohol or the aliphatic aldehyde and the number of carbon atoms in the hydrocarbon group is 8 or more and 36 or less, in an amount of preferably 50% by mass or more, more preferably 70% by mass or more, and further preferably 75% by mass or more.

### [Step (3): Centrifugation Step]

In the centrifugation step of the present invention, the concentrate obtained through the distillation step is separated by a centrifugal force into a precipitate and a supernatant liquid (liquid phase) containing a tertiary amine composition.

The "tertiary amine composition" used herein refers to a composition containing the dialkylmethylamine represented by the formula (I) in an amount of preferably 50% by mass or more, more preferably 70% by mass or more, and further preferably 75% by mass or more.

In the centrifugation step of the present invention, a reaction method is not particularly limited. Examples of the reaction method include a suspension bed method, a fixed-bed batch method, a fixed-bed continuous method, and a combination thereof. In particular, a suspension bed method is preferred from the viewpoint of hue improvement effect of the present invention.

A centrifugation operation that separates the concentrate obtained through the distillation step into a precipitate and a supernatant liquid (liquid phase) is performed under a predetermined centrifugation condition. A centrifugal separator is not particularly limited. Examples of the centrifugal separator include a disk continuous centrifugal separator, a decanter continuous centrifugal separator, and a batch centrifugal separator.

A centrifuge used herein is not particularly limited, and examples of the centrifuge include centrifuges such as "CR22N ROTAR R15A" manufactured by Hitachi, Lid.

### <Centrifugation Condition>

The centrifugal force required for a centrifugation process is preferably 500 G or more, more preferably 1,000 G or more, and further preferably 1,500 G or more, and preferably 30,000 G or less, more preferably 25,000 G or less, and further preferably 20,000 G or less from the viewpoint of suitably removing the colored substance.

The centrifugation time is appropriately adjusted according to the centrifugal force. At a centrifugal force of 500 G or more, the centrifugation time is preferably 20 minutes or more, more preferably 30 minutes or more, and further preferably 60 minutes or more. At a centrifugal force of 1,000 G or more, the centrifugation time is preferably 10 minutes or more, more preferably 15 minutes or more, and further preferably 30 minutes or more. At a centrifugal force of 1,500 G or more, the centrifugation time is preferably 7 minutes or more, more preferably 10 minutes or more, and further preferably 20 minutes or more. Although a longer centrifugation time is more effective, the upper limit of the centrifugation time is 120 minutes or less in terms of efficiency.

As the centrifugation temperature is higher, the viscosity of a liquid is generally lower, and separation is more advantageous. The centrifugation temperature is preferably 5°C or higher, more preferably 10°C or higher, and further preferably 20°C or higher. In terms of operation safety, the centrifugation temperature is preferably 95°C or lower, more preferably 90°C or lower, and further preferably 80°C or lower.

### [Quaternary Ammonium Salt Composition]

The tertiary amine composition obtained by the method according to the present invention can be converted to a quaternary ammonium to obtain a quaternary ammonium salt composition.

A method for converting a tertiary amine composition to a quaternary ammonium to obtain a quaternary ammonium salt composition is not particularly limited, and a publicly known method can be used. For example, the supernatant liquid (liquid phase) collected through the centrifugation step is placed into a container such as a flask, a predetermined amount of quaternizing agent is added to this container, and a mixture is heated at a predetermined temperature for a predetermined time. Thus, the quaternary ammonium salt composition can be obtained.

Examples of the quaternizing agent include an alkyl halide having 1 to 8 carbon atoms (methyl chloride, etc.), a benzyl halide (benzyl chloride, etc.), dialkyl (having 1 to 2 carbon atoms) sulfate (dimethyl sulfate and diethyl sulfate) and a carbonate (dimethyl carbonate, etc.) of an alkyl such as a dialkyl (having 1 to 2 carbon atoms).

### [Tertiary Amine Salt Composition]

The tertiary amine composition obtained by the method according to the present invention can be reacted with an acid to obtain a tertiary amine salt composition.

A method for obtaining a tertiary amine salt composition by a reaction of the tertiary amine composition with an acid is not particularly limited, and a publicly known method can be used. For example, the supernatant liquid (liquid phase) collected through the centrifugation step is placed into a container such as a flask, a predetermined amount of inorganic acid or organic acid is added to this container, and a mixture is heated at a predetermined temperature for a predetermined time. Thus, the tertiary amine salt composition can be obtained.

Examples of the inorganic acid include hydrochloric acid and sulfuric acid.

Examples of the organic acid include monovalent or polyvalent carboxylic acid having 1 to 10 carbon atoms and monovalent or polyvalent sulfonic acid having 1 to 20 carbon atoms. Specific examples thereof include methylsulfonic acid, ethylsulfonic acid, p-toluenesulfonic acid, (o-, m-, or p-)xylenesulfonic acid, benzenesulfonic acid, dodecylbenzenesulfonic acid, glycolic acid, ethylenediamine tetraacetate, citric acid, benzoic acid, salicylic acid, and succinic acid.

### Examples

The present invention will be described in more detail by way of Examples, but the present invention is not limited to the examples. In Examples, a stirring vessel-type reactor including a reaction vessel and a condenser, shown in FIG. 1, was used, and lauryl alcohol and dimethylamine were used as raw materials.

### (Example 1)

### (1) Step (1): Amination Step

Into a 2-L separable flask that was a reaction vessel, 800 g of lauryl alcohol (trade name: KALCOL 2098 available from Kao Corporation) as a raw alcohol was placed, and a powdered Cu/Ni catalyst as a metal catalyst was placed in an amount of 0.3 parts by mass relative to the total amount of the raw alcohol. While the reaction liquid in the reaction vessel was stirred, the inside in the reaction system was replaced by nitrogen.

After the inside in the system was replaced by nitrogen, the supply of hydrogen gas to the reaction vessel was started, and the reaction liquid in the reaction vessel was stirred, heated until the temperature of the reaction liquid was 180°C, and held at this temperature for about 15 minutes. The supply of dimethylamine gas as a raw amine to the reaction vessel was started, and the temperature of the reaction liquid was gradually increased and maintained at 220°C. Thus, an amination was performed.

The supply of the hydrogen gas was performed at a flow rate of 20 L/hr per kilogram of the raw alcohol, the supply of the dimethylamine gas was adjusted according to the progress of the amination such that the flow rate was 10 to 80 L/hr per kilogram of the raw alcohol.

The amination was performed until the amount of unreacted lauryl alcohol in the reaction liquid was 1% by mass or less. The metal catalyst contained in the reaction liquid was removed by filtration to obtain a tertiary amine composition.

### (2) Step (2): Distillation Step

560 mL of the reaction liquid that was a portion of the reaction liquid obtained through the step (1) was placed into a 1-L flask, and a distillation operation was performed under the following distillation condition.

### <Distillation Condition>

• Distillation temperature: 150°C
• Degree of vacuum: 0.6 kPa

By the distillation operation, a distilled product containing a low-boiling-point compound was removed, and 20 g of concentrate in the flask was collected.

### (3) Step (3): Centrifugation Step

Into a 50-mL bottle, 30 g of the concentrate collected by performing the step (2) multiple times was placed. The bottle was put into a centrifuge (trade name: CR22N ROTAR R15A manufactured by Hitachi, Lid.), and a centrifugation operation was performed under a centrifugation condition.

### <Centrifugation Condition>

- Centrifugal force: 1,500 G
- Treatment time: 10 minutes
- Treatment temperature: 22°C

By the centrifugation operation, the concentrate in the bottle was separated into 5 g of precipitate and 25 g of supernatant liquid (liquid phase) containing a tertiary amine composition. The collected liquid phase was subjected to a hue test.

### (Example 2)

The same operation as in Example 1 was performed except that the centrifugal force in the centrifugation condition in the step (3): Centrifugation Step was changed to 3,000 G in Example 1.

### (Example 3)

The same operation as in Example 1 was performed except that the centrifugal force in the centrifugation condition in the step (3): Centrifugation Step was changed to 10,000 G in Example 1.

### (Example 4)

The same operation as in Example 1 was performed except that 5 g of the liquid phase collected through the step (3): Centrifugation Step was placed into a 300-mL flask, 40 g of ethanol and 5 g of 35% by mass hydrochloric acid aqueous solution were added, a mixture was heated at 60°C for 20 minutes, and a tertiary amine hydrochloride composition obtained by a reaction with hydrochloric acid was subjected to a hue test in Example 1.

### (Example 5)

The same operation as in Example 2 was performed except that 5 g of the liquid phase collected through the step (3): Centrifugation Step was placed into a 300-mL flask, 40 g of ethanol and 5 g of 35% by mass hydrochloric acid aqueous solution were added, a mixture was heated at 60°C for 20 minutes, and a tertiary amine hydrochloride composition obtained by a reaction with hydrochloric acid was subjected to a hue test in Example 2.

### (Example 6)

The same operation as in Example 3 was performed except that 5 g of the liquid phase collected through the step (3): Centrifugation Step was placed into a 300-mL flask, 40 g of ethanol and 5 g of 35% by mass hydrochloric acid aqueous solution were added, a mixture was heated at 60°C for 20 minutes, and a tertiary amine hydrochloride composition obtained by a reaction with hydrochloric acid was subjected to a hue test in Example 3.

### (Comparative Example 1)

The same operation as in Example 1 was performed except that the step (3): Centrifugation Step was not performed and the concentrate collected through the step (2): Distillation Step was subjected to a hue test in Example 1.

### (Comparative Example 2)

The same operation as in Example 1 was performed except that the step (3): Centrifugation Step was not performed, the concentrate collected through the step (2): Distillation Step was filtered and separated into a residue and a filtrate under the following filtration condition, and the collected filtrate was subjected to a hue test in Example 1.

### <Filtration Condition>

• Filter paper: quantitative filter paper No. 5C available from ADVANTEC TOYO KAISHA, LTD.

### (Comparative Example 3)

The same operation as in Comparative Example 1 was performed except that 5 g of the concentrate collected through the step (2): Distillation Step was placed into a 300-mL flask, 40 g of ethanol and 5 g of 35% by mass hydrochloric acid aqueous solution were added, a mixture was heated at 60°C for 20 minutes, and a tertiary amine hydrochloride composition obtained by a reaction with hydrochloric acid was subjected to a hue test in Comparative Example 1.

### (Comparative Example 4)

The same operation as in Comparative Example 2 was performed except that 5 g of the filtrate collected by filtration and separation into the residue and the filtrate was placed into a 300-mL flask, 40 g of ethanol and 5 g of 35% by mass hydrochloric acid aqueous solution were added, a mixture was heated at 60°C for 20 minutes, and a tertiary amine hydrochloride composition obtained by a reaction with hydrochloric acid was subjected to a hue test in Comparative Example 2.

### [Measurement by Gas Chromatography]

A portion of the reaction liquid in the 2-L separable flask that was the reaction vessel in the step (1): Amination Step in Example 1 was sampled with time. For the sample, unreacted lauryl alcohol was quantified with respect to an entire peak area of 100% by gas chromatography (trade name: 6500 NETWORK GC manufactured by Agilent Technologies Japan, Ltd.) under the following measurement condition.

### <Measurement condition>

• Column: DB-17HT (0.25 mm in inner diameter × 15 mm in length, 0.15 pm in thickness)
• Oven temperature: 60°C → 320°C
• Detector temperature: 325°C
• Detector: FID

A portion of the concentrate collected through the step (2): Distillation Step in Example 1 was sampled. For the sample, the concentrate composition was qualitatively determined and quantified with an entire peak area of 100% by the same gas chromatography as described above under the same measurement condition as described above. The results are shown in Table 1.

**[Table 1]**

| | Proportion of component in concentrate collected through distillation step (% by mass) |
|---|---|
| Dialkylmethylamine | 80.8 |
| Ester compound | 8.3 |
| Aliphatic alcohol | 0.1 |
| Other component | 10.8 |

### [Hue Test]

The supernatant liquids (liquid phases) collected through the centrifugation step in Examples 1 to 3, the concentrate collected through the distillation step in Comparative Example 1, and the filtrate collected by the filtration in Comparative Example 2 refer to tertiary amine composition samples 1 to 5, respectively. The following hue test was performed. The results are shown in Table 2.

The tertiary amine hydrochloride compositions obtained in Examples 4 to 6 and Comparative Examples 3 and 4 refer to hue indication samples 6 to 10, respectively. The following hue test was performed. The results are shown in Table 3.

### <Degree of Coloration (APHA)>

The Hazen color number (APHA) of each of the samples 1 to 10 was measured using a cell with a length of 1 inch by an ultraviolet-visible spectrophotometer (trade name: Lovibond Tintometer PFX990, manufactured by Tintometer GmbH) in accordance with JIS K 0071-2:1998.

When the value of APHA is smaller, less coloration can be determined.

### <Color Tone>

The color tone of each of the samples 1 to 10 was measured using a cell with a length of 1 inch with respect to red (R), yellow (Y), blue (B), and neutral color (N) by a colorimeter (trade name: Lovibond Tintometer PFX990, manufactured by Tintometer GmbH) in accordance with JIS K 0071-2:1998.

When the value of color tone is smaller, less coloration can be determined.

**[Table 2]**

| Tertiary Amine Composition | | | | | | | | |
|---|---|---|---|---|---|---|---|---|
| | | Centrifugation condition | | Degree of coloration (APHA) | Color tone | | | |
| | | Centrifugal force (G) | Treatment time (min) | | R | Y | B | N |
| Comparative Example 1 | Sample 4 | - | - | 0 | 0.0 | 3.0 | 6.7 | 0.0 |
| Comparative Example 2 | Sample 5 | - | - | 0 | 1.4 | 2.5 | 3.6 | 0.0 |
| Example 1 | Sample 1 | 1,500 | 10 | 0 | 0.1 | 1.0 | 2.5 | 0.0 |
| Example 2 | Sample 2 | 3,000 | 10 | 3 | 0.0 | 1.1 | 1.6 | 0.1 |
| Example 3 | Sample 3 | 10,000 | 10 | 10 | 0.0 | 0.8 | 0.9 | 0.1 |

**[Table 3]**

| Tertiary amine hydrochloride composition | | | | | | | | |
|---|---|---|---|---|---|---|---|---|
| | | Centrifugation condition | | Degree of coloration (APHA) | Color tone | | | |
| | | Centrifugal force (G) | Treatment time (min) | | R | Y | B | N |
| Comparative Example 3 | Sample 9 | - | - | 30 | 2.7 | 4.2 | 3.6 | 0.0 |
| Comparative Example 4 | Sample 10 | - | - | 33 | 1.3 | 0.8 | 0.0 | 0.5 |
| Example 4 | Sample 6 | 1,500 | 10 | 19 | 1.1 | 0.3 | 0.0 | 0.1 |
| Example 5 | Sample 7 | 3,000 | 10 | 16 | 1.1 | 0.3 | 0.0 | 0.1 |
| Example 6 | Sample 8 | 10,000 | 10 | 9 | 1.2 | 0.1 | 0.0 | 0.1 |

### (Summary 1 of Results)

The results of hue test of the tertiary amine compositions shown in Table 2 show as follows.

The hues (in particular, color tones) of the tertiary amine compositions in Examples 1 to 3 are shown to be better than those of the tertiary amine compositions obtained in Comparative Examples 1 and 2 without the centrifugation step. This is because the concentrate obtained through the distillation step defined by the present invention as a target is subjected to the centrifugation step.

The tertiary amine composition in Comparative Example 2 was obtained through the filtration step, but the hue (in particular, color tone) of the tertiary amine composition is shown to be inferior to the tertiary amine compositions in Examples 1 to 3.

### (Summary 2 of Results)

The results of hue test of the tertiary amine hydrochloride compositions shown in Table 3 show as follows.

The hues of the tertiary amine hydrochloride compositions in Examples 4 to 6 are shown to be better than those of the tertiary amine hydrochloride compositions obtained in Comparative Examples 3 and 4 obtained from the tertiary amine compositions obtained without the centrifugation step. This is because the tertiary amine hydrochloride compositions in Examples 4 to 6 are obtained from the tertiary amine composition obtained through the centrifugation step of the concentrate obtained through the distillation step defined by the present invention as a target.

The tertiary amine hydrochloride composition in Comparative Example 4 was obtained from the tertiary amine composition obtained through the filtration step, but the hue of the tertiary amine hydrochloride composition was shown to be inferior to the tertiary amine hydrochloride compositions in Examples 4 to 6.

## Claims

1. A method for producing a tertiary amine composition comprising the following steps (1) to (3):
step (1): an amination step of reacting an aliphatic alcohol or an aliphatic aldehyde having 8 or more and 36 or less carbon atoms with dimethylamine in the presence of a metal catalyst;
step (2): a distillation step of separating a reaction liquid obtained through the step (1) by distillation into a distilled product containing a low-boiling-point compound and a concentrate; and
step (3): a centrifugation step of separating the concentrate obtained through the step (2) by a centrifugal force into a precipitate and a liquid phase containing the tertiary amine composition.

2. The method for producing a tertiary amine composition according to claim 1, wherein in the step (3): the centrifugation step, the centrifugal force is 1,000 G or more and 25,000 G or less.

3. The method for producing a tertiary amine composition according to claim 1 or 2, wherein in the step (2): the distillation step, the concentrate contains 50% by mass or more of dialkylmethylamine represented by a formula (I):
Formula (I): R₂N(CH₃)
wherein R is a hydrocarbon group derived from the aliphatic alcohol or the aliphatic aldehyde, and the number of carbon atoms in the hydrocarbon group is 8 or more and 36 or less.

4. The method for producing a tertiary amine composition according to any one of claims 1 to 3, wherein in the step (2): the distillation step, the concentrate contains 5% by mass or more of ester compound.

5. The method for producing a tertiary amine composition according to any one of claims 1 to 4, wherein in the step (2): the distillation step, the distillation temperature is 70°C or higher and 300°C or lower.

6. The method for producing a tertiary amine composition according to any one of claims 1 to 5, further comprising a filtration step after the step (1): the amination step and before the step (2): the distillation step.

7. The method for producing a tertiary amine composition according to any one of claims 1 to 6, wherein in the step (1): the amination step, the reacting is performed in the presence of hydrogen.

8. The method for producing a tertiary amine composition according to any one of claims 1 to 7, wherein in the step (1): the amination step, the metal catalyst is a catalyst containing at least one selected from the group consisting of copper, nickel, cobalt, iron, ruthenium, platinum, rhodium, palladium, molybdenum, tungsten, and rhenium as a main active component.

9. A quaternary ammonium salt composition obtained by converting a tertiary amine composition obtained by the method for producing a tertiary amine composition according to any one of claims 1 to 8 to a quaternary ammonium.

10. A tertiary amine salt composition obtained by reacting the tertiary amine composition obtained by the method for producing a tertiary amine composition according to any one of claims 1 to 8 with an acid.
